# EUROPEAN PATENT APPLICATION

(11) **EP 0 769 300 A2**
(43) Date of publication of application: **23.04.1997**
(21) Application number: 96307533.8
(22) Date of filing: 17.10.1996
(51) Int. Cl.: A61K 45/06

(54) **Antiemetic composition containing an NK-1 receptor antagonists**

(30) Priority: 20.10.1995 US 5728
(71) Applicant: PFIZER INC., New York, N.Y. 10017 (US)
(72) Inventor: Gonsalves, Susan F., Stonington, Connecticut 06378 (US); Watson, John W., Ledyard, Connecticut 06339 (US); Silberman, Sandra L., Waterford, Connecticut 06385 (US)
(74) Representative: Wilkinson, Stephen John

(57) **Abstract**

The present invention relates to methods of treating or preventing emesis in mammals, including humans, using an NK-1 antagonist in combination with one or more other active agents selected from (a) a glucocorticoid or corticosteroid, (b) a benzodiazepine, (c) metaclopramide and (d) an intracellular molecular scavenger.

## Description

The present invention relates to methods of treating or preventing emesis in mammals, including humans, using an NK-1 antagonist in combination with one or more other active agents selected from (a) a glucocorticoid or corticosteroid, (b) a benzodiazepine, (c) metaclopramide and (d) an intracellular molecular scavenger.

United States Patent 5,393,762, which issued on February 28, 1995 refers to the use of NK-1 receptor antagonists for the treatment of emesis. European Patent Applications 533280A1 and 615751A1, published on March 24, 1993 and September 21, 1994, respectively, also refer to the use of NK-1 receptor antagonists in the treatment of emesis.

United States Patent application 08/353,049, filed December 9, 1994, refers to the use of NK-1 receptor antagonists in combination with 5HT₃ receptor antagonists for the treatment of emesis.

### Summary of the Invention

This invention relates to a method of treating or preventing emesis in a mammal, including a human, comprising administering to such mammal an NK-1 receptor antagonist in combination with one or more other active ingredients selected from (a) a glucocorticoid or corticosteroid, (b) a benzodiazepine, (c) metaclopramide and (d) an intracellular molecular scavenger, wherein the amount of each active ingredient in the combination is such that the combination produces a synergistic antiemetic effect. This method is also referred to herein as "the synergistic combination method".

One embodiment of this invention relates to the above synergistic combination method, wherein there are five active ingredients in the combination and such active ingredients are, respectively, an NK-1 receptor antagonist, a benzodiazepine, a glucocorticoid or corticosteroid, metaclopramide and an intracellular molecular scavenger.

Another embodiment of this invention relates to the above synergistic combination method, wherein there are four active ingredients in the combination and such active ingredients are an NK-1 receptor antagonist and three additional active ingredients selected from (a) a glucocorticoid or corticosteroid, (b) a benzodiazepine (c) metaclopramide and (d) an intracellular molecular scavenger.

Another embodiment of this invention relates to the above synergistic combination method, wherein there are three active ingredients in the combination and such active ingredients are an NK-1 receptor antagonist and two additional active ingredients selected from (a) a glucocorticoid or corticosteroid, (b) a benzodiazepine, (c) metaclopramide and (d) an intracellular molecular scavenger.

Another embodiment of this invention relates to the above synergistic combination method, wherein there are two active ingredients in the combination and such active ingredients are an NK-1 receptor antagonist and one additional active ingredient selected from (a) a glucocorticoid or corticosteroid, (b) a benzodiazepine, (c) metaclopramide and (d) an intracellular molecular scavenger.

Another embodiment of this invention relates to any of the above embodiments of the synergistic combination method wherein no two of the active ingredients that are not NK-1 receptor antagonists in the combination belong to the same one of the following categories (a) through (d): (a) a glucocorticoid or corticosteroid; (b) a benzodiazepine; (c) metaclopramide; and (d) an intracellular molecular scavenger.

Another embodiment of this invention relates to any of the above embodiments of the synergistic combination method wherein one of the active ingredients in the combination is a benzodiazepine selected from lorazepam, medazepam, alprazolam, temazepam, quazepam, triazolam, flunitrazepam, chlordiazepoxide, chlordiazepoxide hydrochloride, clorazepate dipotassium, diazepam, flurazepam, halazepam, oxazepam and prazepam.

Another embodiment of this invention relates to any of the above of the embodiments of the synergistic combination method wherein one of the active ingredients in the combination is a glucocorticoid or corticosteroid selected from dexamethasone, triamcinolone, betamethasone, cortisone, methylprednisolone and hydrocortisone.

Another embodiment of this invention relates to any of the above embodiments of the synergistic combination method wherein one of the active ingredients in the combination is an intracellular molecular scavenger selected from acetylcysteine. glutathione, vitamin D, vitamin E, selenium, and retinoids (e.g., 13-cis-retinoic acid and isotretinoin).

The term "in combination with", as used in this application, means that the NK-1 receptor antagonist and the other active ingredient or ingredients in the combination are administered to the mammal being treated as part of the same therapeutic program according to an appropriate dose regimen prescribed by the treating physician such that the therapeutic effects of such ingredients overlap. The active ingredients may be administered separately or as part of the same pharmaceutical formulation, depending on the particular dosage forms administered and dose regimen prescribed by the treating physician.

Another embodiment of this invention also relates to any of the above embodiments of the synergistic method wherein the NK-1 receptor antagonist administered to said mammal is selected from:
(2S,3S)-3-[2-methoxy-5-(2-thiazolyl)benzyl]amino-2-phenylpiperidine;
(2S,3S)-3-[5-(2-imidazolyl)-2-methoxybenzyl]amino-2-phenylpiperidine;
(2S,3S)-3-[2-methoxy-5-(2-oxopymolidinyl)benzyl]amino-2-phenylpiperidine;
(2S,3S)-3-[2-methoxy-5-(4-methyl-2-thiazolyl)benzyl]-amino-2-phenylpiperidine;
(2S,3S)-3-[2-methoxy-5-(1,2,3-thiadiazol-4-yl)benzyl]-amino-2-phenylpiperidine;
(2S,3S)-(6-methoxy-2-methyl-benzothiazol-5-ylmethyl)-(2-phenylpiperidin-3-yl)amine;
(2S,3S)-[5-(2,5-dimethyl-pyrrol-1-yl)-2-methoxybenzyl]-(2-phenylpiperidin-3-yl)amine;
(2S,3S)-3-[2-methoxy-5-(5-oxazolyl)benzyl]amino-2-phenylpiperidine;
(2S,3S)-(6-methoxy-2-methyl-benzoxazol-5-ylmethyl)-(2-phenyl-pipehdin-3-yl)-amine;
(1SR,2SR,3SR,4RS)-3-[6-methoxy-3-methylbenzisoxazol-5-yl]methylamino-2-benzhydrylazanorbomane;
(2S,3S)-N-(2-methoxy-5-methylsulfonylphenyl)-methyl-2-diphenylmethyl-1-azabicyclo[2.2.2]octan-3-amine;
(2S,3S)-N-(2-methoxy-5-methylthiophenyl)methyl-2-diphenylmethyl-1-azabicyclo[2.2.2]octan-3-amine;
(2S,3S)-N-(2-methoxy-5-dimethylaminophenyl)-methyl-2-diphenylmethyl-1-azabicyclo[2.2.2]octan-3-amine; and
(2S,3S)-N-(5-trifluoroacetylamino-2-methoxyphenyl)methyl-2-diphenylmethyl-1-azabicyclo-[2.2.2]octan-3-amine.
(2S,3S)-3-(5-tert-butyl-2-methoxybenzyl)amino-2-(3-trifluoromethoxyphenyl)piperidine;
(2S,3S)-3-(2-isopropoxy-5-trifluoromethoxybenzyl)amino-2-phenyl-piperidine;
(2S,3S)-3-(2-ethoxy-5-trifluoromethoxybenzyl)amino-2-phenyl-piperidine,
(2S,3S)-3-(2-methoxy-5-trifluoromethoxybenzyl)-amino-2-phenylpiperidine;
(2S,3S)-3(-5-tert-butyl-2-trifluoromethoxybenzyl)amino-2-phenylpiperidine;
2-(diphenylmethyl)-N-(2-methoxy-5-trifluoromethoxy-phenyl)methyl-1-azabicyclo[2.2.2]octan-3-amine;
(2S,3S)-3-[5-chloro-2-(2,2,2-trifluoroethoxy)-benzyl]amino-2-phenylpipendine;
(2S,3S)-3-(5-tert-butyl-2-trifluoromethoxybenzyl)amino-2-phenylpiperidine;
(2S,3S)-3-(2-isopropoxy-5-trifluoromethoxybenzyl)amino-2-phenylpiperidine;
(2S,3S)-3-(2-difluoromethoxy-5-trifluoromethoxybenzyl)-amino-2-phenylpiperidine;
(2S,3S)-2-phenyl-3-[2-(2,2,2-trifluoroethoxybenzyl)-aminopiperidine; and
(2S,3S)-2-phenyl-3-(2-trifluoromethoxybenzyl)]aminopiperidine;
cis-3-(2-chlorobenzylamino)-2-phenylpiperidine;
cis-3-(2-trifluoromethylbenzylamino)-2-phenylpiperidine;
cis-3-(2-methoxybenzylamino)-2-(2-fluorophenyl)piperidine;
cis-3-(2-methoxybenzylamino)-2-(2-chlorophenyl)piperidine;
cis-3-(2-methoxybenzylamino)-2-(2-methylphenyl)piperidine;
cis-3-(2-methoxybenzylamino)-2-(3-methoxyphenyl)piperidine;
cis-3-(2-methoxybenzylamino)-2-(3-fluorophenyl)piperidine;
cis-3-(2-methoxybenzylamino)-2-(3-chlorophenyl)piperidine;
cis-3-(2-methoxybenzylamino)-2-phenylpiperidine;
cis-3-(2-methoxybenzylamino)-2-(3-methylphenyl)piperidine;
cis-3-(2-methoxybenzylamino)-2-(4-fluorophenyl)piperidine;
cis-3-(2-methoxybenzylamino)-2-(3-thienyl)piperidine;
cis-3-(2-methoxybenzylamino)-2-phenylazacyclo-heptane;
3-(2-methoxybenzylamino)-4-methyl-2-phenylpiperidine;
3-(2-methoxybenzylamino)-5-methyl-2-phenylpiperidine;
3-(2-methoxybenzylamino)-6-methyl-2-phenylpiperidine;
(2S,3S)-3-(2-methoxybenzylamino)-2-phenylpiperidine;
(2S,3S)-1-(5-carboethoxypent-1-yl)-3-(2-methoxybenzyl-amino)-2-phenylpiperidine;
(2S,3S)-1-(6-hydroxy-hex-1-yl)-3-(2-methoxybenzyl-amino)-2-phenylpiperidine;
(2S,3S)-1-(4-hydroxy-4-phenylbut-1-yl)-3-(2-methoxy-benzylamino)-2-phenylpiperidine;
(2S,3S)-1-(4-oxo-4-phenylbut-1-yl)-3-(2-methoxybenzyl-amino)-2-phenylpiperidine;
(2S,3S)-1-(5,6-dihydroxyhex-1-yl)-3-(2-methoxybenzyl-amino)-2-phenylpipendine;
cis-3-(5-fluoro-2-methoxybenzylamino)-2-phenylpiperidine;
(2S,3S)-1-[4-(4-fluorophenyl)-4-oxobut-I-yl]-3-(2-methoxybenzylamino)-2-phenylpiperidine;
(2S,3S)-1-[4-(4-fluornphenyl)-4-hydroxybut-1-yl]-3-(2-methoxybenzylamino)-2-phenylpiperidine;
cis-3-(2-methoxy-5-methylbenzylamino)-2-phenylpiperidine;
(2S,3S)-1-(4-benzamidobut-1-yl)-3-(2-methoxybenzyl-amino)-2-phenylpiperidine;
cis-3-(2-methoxynaphth-1-ylmethylamino)-2-phenylpiperidine;
(2S,3S)-3-(2-methoxybenzylamino)-1-(5-N-methyl-carboxamidopent-1-yl)-2-phenylpiperidine;
(2S,3S)-1(4-cyanobut-1-yl)-3-(2-methoxybenzylamino)-2-phenylpiperidine;
(2S,3S)-1-[4-(2-naphthamido)but-1-yl]-3-(2-methoxy-benzylamino)-2-phenylpiperidine;
(2S,3S)-1-(5-benzamidopent-1-yl)-3-(2-methoxybenzyl-amino)-2-phenylpiperidine;
(2S,3S)-1-(5-aminopent-1-yl)-3-(2-methoxybenzylamino)-2-phenylpiperidine;
(2S,3S)-3-(5-chloro-2-methoxybenzylamino)-2-phenylpipendine;
(2S,3S)-3-(2,5-dimethoxybenzylamino)-2-phenylpiperidine;
cis-3-(3,5-difluoro-2-methoxybenzylamino)-2-phenylpiperidine;
cis-3-(4,5-difluoro-2-methoxybenzylamino)-2-phenylpiperidine;
cis-3-(2,5-dimethoxybenzylamino)-1-[4-(4-fluorophenyl)-4-oxobut-1-yl]-2-phenylpiperidine;
cis-3-(5-chloro-2-methoxybenzylamino)-1-(5,6-dihydroxyhex-1-yl)-2-phenylpiperidine;
cis-1-(5,6-dihydroxyhex-1-yl)-3-(2,5-dimethoxy-benzylamino)-2-phenylpiperidine;
cis-2-phenyl-3-[-2(prop-2-yloxy)benzylamino]piperidine;
cis-3-(2,5-dimethoxybenzyl)amino-2-(3-methoxy-phenyl)piperidine hydrochloride;
cis-3-(5-chloro-2-methoxybenzyl)amino-2-(3-methoxy-phenyl)piperidine dihydrochloride;
cis-3-(5-chloro-2-methoxybenzyl)amino-2-(3-chloro-phenyl)piperidine dihydrochloride;
3-(2-methoxybenzylamino)-2,4-diphenylpipendine;
cis-3-(2-methoxybenzylamino)-2-phenylpyrrolidine;
(2S,3S)-3-(5-ethyl-2-methoxybenzyl)amino-2-phenylpiperidine;
(2S,3S)-3-(5-n-butyl-2-methoxybenzyl)amino-2-phenylpiperidine;
(2S,3S)-3-(2-methoxy-5-n-propylbenzyl)amino-2-phenylpiperidine;
(2S,3S)-3-(5-isopropyl-2-methoxybenzyl)amino-2-phenylpiperidine;
(2S,3S)-3-(5-s-butyl-2-methoxybenzyl)amino-2-phenylpiperidine;
(2S,3S)-3-(5-t-butyl-2-methoxybenzyl)amino-2-phenylpiperidine;
(2S,3S)-3-(2-methoxy-5-phenylbenzyl)amino-2-phenylpiperidine;
(2S,3S)-N-(5-isopropyl-2-methoxyphenyl)methyl-2-diphenylmethyl-1-azabicyclo-[2.2.2]octan-3-amine;
(2S,3S)-N-(5-tert-butyl-2-methoxyphenyl)methyl-2-diphenylmethyl-1-azabicyclo[2.2.2]octan-3-amine;
(2S,3S)-N-(5-methyl-2-methoxyphenyl)methyl-2-diphenylmethyl-1-azabicyclo[2.2.2]octan-3-amine;
(2S,3S)-N-(5-ethyl-2-methoxyphenyl)methyl-2-diphenylmethyl-1-azabicyclo[2.2.2]octan-3-amine;
(2S,3S)-N-(5-isopropyl-2-methoxyphenyl)methyl-2-diphenylmethyl-1-azabicyclo[2.2.2]octan-3-amine;
(2S,3S)-N-(5-sec-butyl-2-methoxyphenyl)methyl-2-diphenylmethyl-1-azabicyclo(2.2.2]octan-3-amine; and
(2S,3S)-N-(5-n-propyl-2-methoxyphenyl)methyl-2-diphenylmethyl-1-azabicyclo[2.2.2]octan-3-amine;
2,4-dimethylthiazole-5-sulfonic acid[4-methoxy-3-((2S,3S)-2-phenylpiperidin-3-ylaminomethyl)phenyl]-methylamide;
N-(4,5-dimethylthiazol-2-yl)-N-[4-methoxy-3-((2S,3S)-2-phenylpiperidin-3-yl-aminomethyl)phenyl]-methanesulfonamide;
{5-[(4,5-dimethylthiazol-2-yl)methylamino]-2-methoxybenzyl)-((2S,3S)-2-phenylpiperidin-3-yl)amine;
{5-(4,5-dimethylthiazol-2-ylamin o)-2-methoxybenzyl}-((2S,3S)-2-phenylpiperidin-3-ylamine;
4,5-dimethylthiazole-2-sulfonic acid methyl-[3-((2S,3S)-2-phenylpipendin-3-ylaminomethyl)-4-trifluoromethoxyphenyl]-amide;
2,4-dimethylthiazole-5-sulfonicacid[4-isopropoxy-3-((2S,3S)-2-phenylpiperidin-3-ylaminomethyl)phenyl]-methylamide;
2,4-dimethylthiazole-5-sulfonicacid[4-isopropoxy-3-((2S,3S)-2-phenylpiperidin-3-ylaminomethyl)phenyl]-isopropylamide;
2,4-dimethylthiazole-5-sulfonic acid [4-methoxy-3-((2S,3S)-2-phenylpiperidin-3-ylaminomethyl)phenyl]-isopropylamide;
2,4dimethylthiazole-5-sulfonic acid(4-methoxy-3-((2S,3S)-2-phenylpiperidin-3-ylaminomethyl)phenyl]-isobutylamide;
2,4-dimethylthiazole-5-sulfonicacid[4-isopropoxy-3-((2S,3S)-2-phenylpipendin-3-ylaminomethyl)phenyl]-isobutylamide;
(3R,4S,5S,6S)-N,N-diethyl-5-(5-isopropyl-2-methoxy-benzylamino)-6-diphenylmethyl-1-azabicyclo(2.2.2]octane-3-carboxamide;
(3R,4S,5S,6S)-N,N-diethyl-5-(2,5-dimethoxybenzylamino)-6-diphenylmethyl-1-azabicyclo[2.2.2]octane-3-carboxamide;
(3R,4S,5S,6S)-5-(5-isopropyl-2-methoxybenzylamino)-6-diphenylmethyl-1-azabicyclo[2.2.2]octane-3-carboxylic acid;
(3R,4S,5S,6S)-5-(2-methoxy-2-methylthiobenzylamino)-6-diphenylmethyl-1-azabicyclo[2.2.2]octane-3-carboxylic acid;
(3R,4S,5S,6S)-5-(2,5-dimethoxybenzylamino)-6-diphenyl-methyl-1-azabicyclo-[2.2.2]octane-3-carboxylic acid;
(3R,4S,5S,6S)-5-(2-methoxy-5-methylbenzylamino)-6-diphenylmethyl-1-azabicyclo[2.2.2]octane-3-carboxylic acid;
(3R,4S,5S,6S)-5-(5-ethyl-2-methoxybenzylamino)-6-diphenylmethyl-1-azabicyclo[2.2.2]octane-3-carboxylic acid;
(3R,4S,5S,6S)-5-(2-methoxyl-5-n-propylbenzylamino)-6-diphenylmethyl-1-azabicyclo[2.2.2]octane-3-carboxylic acid;
(3R,4S,5S,6S)-5-(5-sec-butyl-2-methoxybenzylamino)-6-diphenylmethyl-1-azabicyclo[2.2.2]octane-3-carboxylic acid;
(3R,4S,5S,6S)-5-(5-N-methyl-methanesulfonylamino-2-methoxy-benzylamino)-6-diphenylmethyl-1-azabicyclo(2.2.2]octane-3-carboxylic acid;
(3R,4S,5S,6S)-5-(2-methoxy-5-methylsulf inylbenzyl-amino)-6-diphenylmethyl-1-azabicyclo[2.2.2]octane-3-carboxylic acid;
(3R,4S,5S,6S)-5-(2-methoxy-5-trifluoromethoxybenzyl-amino)-6-diphenylmethyl-1-azabicyclo[2.2.2]octane-3-carboxylic acid;
(3R,4S,5S,6S)-5-(2-methoxy-5-methylsulfonylbenzyl-amino)-6-diphenylmethyl-1-azabicyclo[2.2.2]octane-3-carboxylic acid;
(3R,4S,5S,6S)-5-(5-dimethylamino-2-methoxybenzylamino)-6-diphenylmethyl-1-azabicyclo[2.2.2]octane-3-carboxylic acid;
(3R,4S,5S,6S)-5-(5-isopropyl-2-methoxybenzylamino)-6-diphenylmethyl-1-azabicyclo[2.2.2]octane-2-carboxylic acid;
(3R,4S,5S,6S)-5-(2-methoxy-5-methylthiobenzylamino)-6-diphenylmethyl-1-azabicyclo[2.2.2]octane-2-carboxylic acid;
(3R,4S,5S,6S)-5-(2,5-dimethoxybenzylamino)-6-diphenylmethyl-1-azabicyclo[2.2.2]octane-2-carboxylic acid;
(3R,4S,5S,6S)-5-(2-methoxy-5-methylbenzylamino)-6-diphenylmethyl-1-azabicyclo[2.2.2]octane-2-carboxylic acid;
(3R,4S,5S,6S)-5-(5-ethyl-2-methoxybenzylamino)-6-diphenylmethyl-1-azabicyclo[2.2.2]octane-2-carboxylic acid;
(3R,4S,5S,6S)-5-(2-methoxyl-5-n-propylbenzylamino)-6-diPhenylmethyl-1-azabicyclo[2.2.2]octane-2-carboxylic acid;
(3R,4S,5S,6S)-5-(5-sec.butyl-2-methoxybenzylamino)-6-diphenylmethyl-1-azabicyclo[2.2.2]octane-2-carboxylic acid;
(3R,4S,5S,6S)-5-(5-N-methylmethanesulfonylamino-2-methoxybenzyl-amino)-6-diphenylmethyl-1-azabicyclo[2.2.2]octane-2-carboxylic acid;
(3R,4S,5S,6S)-5-(2-methoxy-5-methylsulfinylbenzylamino)6-diphenylmethyl-1-azabicyclo[2.2.2]octane-2-carboxylic acid;
(3R,4S,5S,6S)-5-(2-methoxy-5-trifluoromethoxybenzylamino)-6-diphenylmethyl-1-azabicyclo[2.2.2]octane-2-carboxylic acid;
(3R,4S,5S,6S)-5-(2-methoxy-5-methylsulfonylbenzyl-amino)-6-diphenylmethyl-1-azabicyclo[2.2.2]octane-2-carboxylic acid;
(3R,4S,5S,6S)-5-(5-dimethylamino-2-methoxybenzylamino)-6-diphenylmethyl-1-azabicyclo[2.2.2]octane-2-carboxylic acid;
(3R,4S,5S,6S)-N-carbamoylmethyl-5-(5-isopropyl-2-methoxybenzylamino)-6-diphenylmethyl-1-azabicyclo[2.2.2]octane-3-carboxamide;
(3R,4S,5S,6S)-N-carboxymethyl-5-(5-isopropyl-2-methoxybenzylamino)-6-diphenylmethyl-1-azabicyclo[2.2.2]octane-3-carboxamide;
(3R,4S,5S,6S)-3-(2-carbamoylpyrrolidin-1-yl)carbonyl-5-(5-isopropyl-2-methoxybenzylamino)-6-diphenylmethyl-1-azabicyclo[2.2.2]octane;
(3R*,4S*,5S*,6S*)-N-(1-carbamoylethyl)-5-(5-isopropyl-2-methoxybenzylamino)-6-diphenylmethyl-1-azabicyclo[2.2.2]octane-3-carboxamide;
(3R,4S,5S,6S)-N-(1-(carbamoyl-3-methylbutyl)-5-(5-isopropyl-2-methoxybenzylamino)-6-diphenylmethyl-1-azabicyclo[2.2.2]octane-3-carboxamide;
(3R,4S,5S,6S)-N-(2-carbamoylethyl)-5-(5-isopropyl-2-methoxybenzylamino)-6-diphenylmethyl-1-azabicyclo[2.2.2]octane-3-carboxamide;
(2S,3S)-N-(5-lsopropenyl-2-methoxyphenyl)methyl-2-diphenylmethyl-1-azabicyclo[2.2.2]octan-3-amine;
(2S,3S)-N-(2-methoxy-5-vinylphenyl)methyl-2-diphenylmethyl-1-azabicyclo[2.2.2]octan-3-amine;
(2S,3S)-N-(2-methoxy-4,5-dimethylphenyl)methyl-2-diphenylmethyl-1-azabicyclo[2.2.2]octan-3-amine;
(2S,3S)-N-(5,6,7,8-tetrahydro-3-methoxy-2-naphthyl)methyl-2-diphenylmethyl-1-azabicyclo[2.2.2]octan-3-amine;
(2S,3S)-N-(5-methoxyindan-6-yl)methyl-6-diphenylmethyl-1-azabicyclo[2.2.2]octan-3-amine;
(2S,3S)-3-(2,4-dimethoxy-5-ethylbenzylamino)-2-diphenylmethyl-1-azabicyclo[2.2.2.]octane;
(2S,3S)-2-diphenylmethyl-N-(2-methoxy-5-diethylphosphoryl)phenyl]methyl-1-azabicyclo[2.2.2]octan-3-amine;
(3R,4S,5S,6S)-5-(5-isopropenyl-2-methoxybenzyl-amino)-6-diphenylmethyl-1-azabicyclo[2.2.2]octane-3-carboxamide;
(3R,4S,5S,6S)-6-diphenylmethyl-5-(2-methoxy-5-methylsulfonylbenzylamino)-1-azabicyclo[2.2.2]octane-3-carboxamide;
(3R,4S,5S,6S)-5-[5-(N-acetyl-N-methylamino)-2-methoxybenzylamino]-6-diphenylmethyl-1-azabicyclo[2.2.2]octane-3-carboxamide;
(3R,4S,5S,6S)-6-diphenylmethyl-5-[2-methoxy-5-(N-methyl-N-methylsulfonylamino)benzylamino]-1-azabicyclo[2.2.2]octane-3-carboxamide;
(3R,4S,5S,6S)-6-diphenylmethyl-5-(2-methoxy-5-methylsulfonylbenzylamino)-1-azabicyclo[2.2.2]octane-3-carboxylic acid;
(2S,3S)-N-[5-(1-hydroxy-1-methylethyl)-2-methoxy-phenyl]methyl-2-diphenylmethyl-1-azabicyclo[2.2.2]octan-3-amine;
(2S,3S)-N-[2-methoxy-5-(1-methoxy-1-methylethyl)-phenyl]methyl-2-diphenylmethyl-1-azabicyclo[2.2.2]octan-3-amine;
(3R,4S,5S,6S)-3-[5-(1-hydroxy-1-methylethyl)-2-methoxyphenyl]methylamino-6-diphenylmethyl-1-azabicyclo[2.2.2]octan-5-carboxylic acid;
(2S,3S)-2-diphenylmethyl-N-[5-(1-hydroxy-1-hydroxymethylethyl)-2-methoxyphenyl]methyl-1 -azabicyclo[2.2.2]octan-3-amine;
(3R,4S,5S,6S)-3-[5-(1-methoxy-1-methylethyl)-2-methoxyphenyl]methylamino-6-diphenylmethyl-1-azabicyclo[2.2.2]octan-5-carboxylic acid;
(3R,4S,5S,6S)-3-[5-(1-hydroxy-1-methylethyl)-2-methoxyphenyl]methylamino-6-diphenylmethyl-1-azabicyclo[2.2.2]octan-5-carboxylic acid;
(3R,4S,5S,6S)-3-(5-(1-ethylthio-1-methylethyl)-2-methoxyphenylimethylamino-6-diphenylmethyl-1-azabicyclo[2.2.2]octan-5-carboxylic acid;
(3S,4R,5S,6S)-N-carbamoylmethyl-6-diphenylmethyl-5-(3,5-bis(trifluoromethyl)benzyloxy)-1-azabicyclo[2.2.2] octane-3-carboxamide;
(3S,4R,5S,6S)-6-diphenylmethyl-5-(3,5-bis(trifluoromethyl)benzyloxy)-1-azabicyclo[2.2.2]octane-3-carboxamide;
(3S,4R,5S,6S)-N,N-(3-oxa-1,5-pentylene)-6-diphenylmethyl-5-(3,5-bis(trifluoromethyl)benzyloxy)-1-azabicyclo(2.2.2]octane-3-carboxamide;
(3S,4R,5S,6S)-6-diphenylmethyl-5-(3,5-dimethylbenzyloxy)-1-azabicyclo[2.2.2]octane-3-carboxylic acid;
(3S,4R,5S,6S)-N,N-diethyl-6-diphenylmethyl-5-(3,5-bis(trifluoromethyl)benzyloxy)-1-azabicyclo[2.2.2]octane-3-carboxamide;
(3S,4R,5S,6S)-6-djphenylmethyl-5-(3-fluoro-5-trifluoromethylbenzyloxy)-1-azabicyclo[2.2.2]octane-3-carboxylic acid;
(35,4R,5S,6S)-6-diphenylmethyl-5-(3,5-bis(trifluoromethyl)benzyloxy)-1-azabicyclo[2.2.2]octane-3-carboxylic acid:
(3S,4R,5S,6S)-N,N-dimethyl-6-diphenylmethyl-5-(3,5-bis(trifluoromethyl)benzyloxy)-1-azabicyclo[2.2.2]octane-3-carboxamide;
(2S*,3S*,4S*,5S*)-4-carboxy-3-[N-(5-isopropyl-2-methoxybenzyl)amino]-5-methyl-2-phenylpyrrolidine;
(2S*,3S*,5S*)-5-carboxy-3-[N-(2-methoxy-5-trifluoromethoxybenzyl)amino]-2-phenylpiperidine;
(2S,3S)-3-(6-methoxy-1-methyl-2-oxo-1,2,3,4-tetrahydroquinolin-7-yl)methyl-2-phenylpiperidine-3-amine;
(2S,3S)-2-phenyl-N-[5-[2,2,2-trifluoro-1-(trifluoromethyl)ethyl]-2-methoxybenzyl]piperidine-3-amine;
(2S,3S)-2-diphenylmethyl-N-[2-methoxy-5-(methylsulfonyl)benzyl]-1-azabicyclo[2.2.2]ctane-3-amine;
(2S,3S)-2-diphenylmethyl-N-(5-isopropenyl-2-methoxybenzyl)-1-azabicyclo[2.2.2]octane-3-amine; and
(2S,3S)-2-diphenylmethyl-N-(5-(1-hydroxy-1-methylethyl)-2-methoxybenzyl]-1-azabicyclo[2.2.2]octane-3-amine.

The above NK-1 receptor antagonists that are basic in nature are capable of forming a wide variety of different salts with various inorganic and organic acids. Examples of acids that form suitable pharmaceutically acceptable salts for use in this invention are those that form non-toxic acid addition salts, i.e., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [i.e., 1,1'-methylene-bis-(2-hydroxy-3-naphthoate)]salts.

Those NK-1 antagonists that are also acidic in nature are capable of forming base salts with various pharmacologically acceptable cations. The chemical bases that are used as reagents to prepare the pharmaceutically acceptable base salts of the therapeutic agents are those that form non-toxic base salts with the acidic therapeutic agents. Such non-toxic base salts include those derived from such pharmacologically acceptable cations as sodium, potassium, calcium and magnesium, etc.

The treatment of emesis includes the treatment of nausea, retching and vomiting. Emesis includes acute emesis, delayed emesis and anticipatory emesis. Substance P receptor antagonists are useful in the treatment of emesis, however induced. For example, emesis may be induced by drugs such as cancer chemotherapeutic agents (e.g., cyclophosphamide, carmustine, lomustine and chlorambucil), cytotoxic antibiotics (e.g., dactinomycin, doxorubicin, mitomycin-C and bleomycin), opioid analgesics (e.g., morphine), anti-metabolites (e.g., cytarabine, methotrexate and 5-fluorouracil), vinca alkaloids (e.g., etoposide, vinblastine and vincristine), and other drugs such as cisplatin, dacarbazine, procarbazine and hydroxyurea. Emesis may also be induced by radiation sickness, radiation therapy, poisons, toxins such as those caused by metabolic disorders or by infection (e.g., gastritis), pregnancy, vestibular disorders such as motion sickness, post-operative sickness, gastrointestinal obstruction, reduced gastrointestinal motility, visceral pain (e.g., myocardial infarction or peritonitis), migraine, increased intercranial pressure or decreased intercranial pressure (e.g., altitude sickness).

The methods of this invention may also be used to treat or prevent emesis induced by the drug ipecac.

### Detailed Description Of The Invention

The following references refer, collectively, to quinuclidine, piperidine, ethylene diamine, pyrrolidine and azanorbomane derivatives and related compounds that exhibit activity as NK-1 receptor antagonists and which can be used, in combination with from one to four other active ingredients, as described above, in the pharmaceutical compositions and methods of this invention, and to methods of preparing the same: United States Patent 5,162,339, which issued on November 11, 1992; United States Patent 5,232,929, which issued on August 3, 1993; World Patent Application WO 92/20676, published November 26, 1992; World Patent Application WO 93/00331. published January 7, 1993; World Patent Application WO 92/21677, published December 10, 1992; World Patent Application WO 93/00330, published January 7. 1993; World Patent Application WO 93/06099, published April 1, 1993; World Patent Application WO 93/10073, published May 27, 1993; World Patent Application WO 92/06079, published April 16, 1992; World Patent Application WO 92/12151, published July 23, 1992; World Patent Application WO 92/15585, published September 17, 1992. World Patent Application WO 93/10073, published May 27, 1993; World Patent Application WO 93/19064, published September 30, 1993; World Patent Application WO 94/08997, published April 28, 1994; World Patent Application WO 94/04496, published March 3, 1994; World Patent Application WO 94/13663, published June 23, 1994; World Patent Application WO 94/20500, published September 15, 1994; World Patent Application PCT/IB94/00221, which designates the United States and was filed on July 18, 1994; World Patent Application PCT/JP94/00781, which designates the United States and was filed on May 13, 1994; World Patent Application PCT/JP94/01092, which designates the United States and was filed on July 5, 1994; and World Patent Application PCT/JP94/01514, which designates the United States and was filed on September 13, 1994. All of the foregoing World Patent Applications designate the United States as a national state in which they will be prosecuted. The foregoing patents and patent applications are incorporated herein by reference in their entireties.

The specific NK-1 receptor antagonists listed in the Summary of the Invention can be prepared by methods described in the patents and patent applications listed above and in the scientific literature.

Other NK-1 receptor antagonists that can be used in the combination therapies of this invention are described in the following references: European Patent Application EP 499,313, published August 19, 1992; European Patent Application EP 520,555, published December 30, 1992; European Patent Application EP 522,808, published January 13, 1993, European Patent Application EP 528,495, published February 24, 1993, PCT Patent Application WO 93/14084, published July 22, 1993, PCT Patent Application WO 93/01169, published January 21, 1993, PCT Patent Application WO 93/01165, published January 21, 1993, PCT Patent Application WO 93/01159, published January 21, 1993, PCT Patent Application WO 92/20661, published November 26, 1992, European Patent Application EP 517,589, published December 12, 1992, European Patent Application EP 428,434, published May 22, 1991, European Patent Application EP 360,390, published March 28, 1990, PCT Patent Application WO 95/04042, published February 9, 1995, PCT Patent Application WO 95/08549, published March 30, 1995, PCT Patent Application WO 95/19344, published July 20, 1995, PCT Patent Application WO 95/23810, published September 8, 1995, and PCT Patent Application WO 95/20575, published August 3, 1995. These publications are also incorporated herein by reference in their entireties.

This invention relates both to methods of treating emesis in which the NK-1 receptor antagonist and one or more of the other active ingredients are administered together, as part of the same pharmaceutical composition, as well as methods in which they are administered separately as part of an appropriate dose regimen designed to obtain the benefits of the combination therapy. The appropriate dose regimen, the amount of each dose administered, and specific intervals between doses of each active agent will depend upon the specific combination of active agents employed, the condition of the patient being treated, the emetogen and the severity of the condition.

The active agents used in the synergistic combination method of this invention will be administered in amounts so that the combination of active agents produces a synergistic effect. They will generally be administered in amounts less than or equal to those for which they are effective as single agents for the treatment or prevention of emesis, and, for active agents that have not been used as antiemetic agents, those for which they are administered to mammals for any therapeutic purpose. The FDA approved dosages for those active agents used in the synergistic method of the invention that have received FDA approval for administration to humans are publicly available (see, for example, Remington's Pharmaceutical Sciences, 18th Edition, Mack Publishing Co., Easton, PA, 1990, and Physician's Desk Reference, 49th Edition, Medical Economics Data Production Co., Montvale, NJ, 1995). Similarly, the dose regimen for each of the active ingredients in the combination will be prescribed by the treating physician, taking into account the factors listed above. Such regimens will generally be similar to those known to be effective for the same active agents when used as single agent anti-emetics or as single components in antiemetic combination therapy.

Examples of single agent antiemetic dosages that have been reported in the literature for certain of the active agents that can be employed in the methods of this invention are stated below.
- dexamethasone:: 4-20 mg per dose (po/iv), once only or every 4-6 hours
- lorazepam:: 1.0-3.0 mg/m² per dose (iv), once only or every 6 hours
- metaclopramide:: 1-3 mg/kg (iv) per dose, every 2 hours for 2-5 doses
(See Pisters et al., "Management of Nausea and Vomiting Caused by Anticancer Drugs: State of the Art", Oncology, 6, pp. 107-112, 1992). The following articles also refer to antiemetic dosages of certain of the active agents that can be used in the synergistic combination method of this invention: Short, R., "Antiemetic Claims for Glutathione Backed by Phase III Trial", INPHARMA, Jan. 21, 1995; Borgeat et al., "Preliminary Communication: Adjuvant Propofol Enables Better Control of Nausea and Emesis . . . ," Canadian Journal of Anesthesia,1994, 41 (11), pp 1117-9; "Drugs For Vomiting Caused by Cancer Chemotherapy", The Medical Letter, 35, pp. 124-6, 1993, The Medical Letter, Inc., New Rochelle, NY; Cerosimo et al., "Adrenal Corticosteroids As Antiemetics During Cancer Chemotherapy", Pharmacotherapy, 6, 1986, pp. 118-127; Triozzi et al., "Optimum Management of Nausea and Vomiting in Cancer Chemotherapy", Drugs, 34, pp 136-149 (1987); and Grunberg et al., "Control of Chemotherapy Induced Emesis", New England Joumal of Medicine, Dec. 9, 1993, pp. 1790-1796. The foregoing articles are incorporated herein by reference in their entireties.

Generally, in carrying out the synergistic combination method of this invention, the NK-1 receptor antagonist will be administered to an average 70 kg adult human in an amount ranging from about 0.36 to about 8.6 mg per kg body weight of the subject being treated per day, in single or divided doses, preferably from about 0.36 to about 4.3 mg/kg. Variations may nevertheless occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effect, provided that such larger doses are first divided into several small doses for administration throughout the day.

The combination of active agents used in the synergistic combination method of this invention can also be used for the treatment of emesis in further combination with a 5HT₃ receptor antagonist (e.g., ondansetron, granisetron or tropisetron). The NK-1 receptor antagonists and the other active ingredients that are

employed in the synergistic combination method of this invention are hereinafter also referred to as "therapeutic agents". The therapeutic agents can be administered via either the oral or parenteral route. They will generally be administered orally or parenterally daily, in single or divided doses. so that the total amount of each active agent administered falls within the above guidelines.

The therapeutic agents may be administered alone or in combination with pharmaceutically acceptable carriers or diluents by either of the routes previously indicated, and such administration may be carried out in single or multiple doses. More particularly, the therapeutic agents of this invention can be administered in a wide variety of different dosage forms, i.e., they may be combined with various pharmaceutically acceptable inert carriers in the form of tablets, capsules, lozenges, troches, hard candies, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the therapeutic compounds of this invention, when administered separately (i.e., not in the same pharmaceutical composition) are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably com, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a therapeutic agent in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

The activity of certain therapeutic agents as substance P receptor antagonists may be determined by their ability to inhibit the binding of substance P at its receptor sites in bovine caudate tissue, employing radioactive ligands to visualize the tachykinin receptors by means of autoradiography. The substance P antagonizing activity of the herein described compounds may be evaluated by using the standard assay procedure described by M. A. Cascieri et al., as reported in the Journal of Biological Chemistry, Vol. 258, p. 5158 (1983). This method essentially involves determining the concentration of the individual compound required to reduce by 50% the amount of radiolabelled substance P ligands at their receptor sites in said isolated cow tissues, thereby affording characteristic IC₅₀ values for each compound tested.

In this procedure, bovine caudate tissue is removed from a -70°C freezer and homogenized in 50 volumes (w./v.) of an ice-cold 50 mM Tris (i.e., trimethamine which is 2-amino-2-hydroxymethyl-1,3-propanediol) hydrochloride buffer having a pH of 7.7. The homogenate is centrifuged at 30,000 x G for a period of 20 minutes. The pellet is resuspended in 50 volumes of Tris buffer, rehomogenized and then recentrifuged at 30,000 x G for another twenty-minute period. The pellet is then resuspended in 40 volumes of ice-cold 50 mM Tris buffer (pH 7.7) containing 2 mM of calcium chloride, 2 mM of magnesium chloride, 4 µg/ml of bacitracin, 4µg/ml of leupeptin, 2µg of chymostatin and 200 µg/ml of bovine serum albumin. This step completes the production of the tissue preparation.

The radioligand binding procedure is then carried out in the following manner, viz., by initiating the reaction via the addition of 100 µl of the test compound made up to a concentration of 1 µM, followed by the addition of 100 µl of radioactive ligand made up to a final concentration 0.5 mM and then finally by the addition of 800 µl of the tissue preparation produced as described above. The final volume is thus 1.0 ml, and the reaction mixture is next vortexed and incubated at room temperature (ca. 20°C) for a period of 20 minutes. The tubes are then filtered using a cell harvester, and the glass fiber filters (Whatman GF/B) are washed four times with 50 mM of Tris buffer (pH 7.7), with the filters having previously been presoaked for a period of two hours prior to the filtering procedure Radioactivity is then determined in a Beta counter at 53% counting efficiency, and the IC₅₀ values are calculated by using standard statistical methods.

## Claims

1. The use of an NK-1 receptor antagonist in the preparation of a medicament for use in combination with one or more other active ingredients selected from (a) a glucocorticoid or corticosteroid, (b) a benzodiazepine, (c) metaclopramide and (d) an intracellular molecular scavenger in the treatment or prevention of emesis in a mammal.

2. The use of an NK-1 receptor antagonist in combination with one or more other active ingredients selected from (a) a glucocorticoid or corticosteroid, (b) a benzodiazepine, (c) metaclopramide and (d) an intracellular molecular scavenger in the preparation of a medicament for use in the treatment or prevention of emesis in a mammal.

3. The use according to claim 1 or claim 2, wherein there are five active ingredients in the combination and such active ingredients are, respectively, an NK-1 receptor antagonist, a benzodiazepine, a glucocorticoid or corticosteroid, metaclopramide and an intracellular molecular scavenger.

4. The use according to claim 1 or claim 2, wherein there are four active ingredients in the combination and such active ingredients are an NK-1 receptor antagonist and three additional active ingredients selected from (a) a glucocorticoid or corticosteroid, (b) a benzodiazepine, (c) metaclopramide and (d) an intracellular molecular scavenger.

5. The use according to claim 1 or claim 2, wherein there are three active ingredients in the combination and such active ingredients are an NK-1 receptor antagonist and two additional active ingredients selected from (a) a glucocorticoid or corticosteroid, (b) a benzodiazepine, (c) metaclopramide and (d) an intracellular molecular scavenger.

6. The use according to claim 1 or claim 2, wherein there are two active ingredients in the combination and such active ingredients are an NK-1 receptor antagonist and one additional active ingredient selected from (a) a glucocorticoid or corticosteroid, (b) a benzodiazepine, (c) metaclopramide and (d) an intracellular molecular scavenger.

7. The use according to claim 1 or claim 2, wherein no two of the active ingredients that are not NK-1 receptor antagonists in the combination belong to the same one of the following categories (a) through (d): (a) a glucocorticoid or corticosteroid, (b) a benzodiazepine, (c) metaclopramide, and (d) an intracellular molecular scavenger.

8. The use according to claim 1 or claim 2, wherein one of the active ingredients in the combination is a benzodiazepine selected from lorazepam, medazepam, alprazolam, temazepam, quazepam, triazolam, flunitrazepam, chlordiazepoxide, chlordiazepoxide hydrochloride, clorazepate dipotassium, diazepam, flurazepam, halazepam, oxazepam and prazepam.

9. The use according to claim 1 or claim 2, wherein one of the active ingredients in the combination is a glucocorticoid or corticosteroid selected from dexamethasone, triamcinolone, betamethasone, cortisone, methylprednisolone and hydrocortisone.

10. The use according to claim 1 or claim 2, wherein one of the active ingredients in the combination is an intracellular molecular scavenger selected from acetylcysteine, vitamin D, vitamin E, selenium, and retinoids (e.g., 13-cis-retinoic acid and isotretinoin).

11. The use according to claim 1 or claim 2, wherein the NK-1 receptor antagonist administered to said mammal is selected from:
(2S,3S)-3-(2-methoxy-5-(2-thiazolyl)benzyl]amino-2-phenylpiperidine;
(2S,3S)-3-[5-(2-imidazolyl)-2-methoxybenzyl]amino-2-phenylpiperidine;
(2S,3S)-3-[2-methoxy-5-(2-oxopyrrolidinyl)benzyl]amino-2-phenylpipeddine;
(2S,3S)-3-[2-methoxy-5-(4-methyl-2-thiazolyl)benzyl]-amino-2-phenylpiperidine;
(2S,3S)-3-[2-methoxy-5-(1,2,3-thiadiazol-4-yl)benzyl]-amino-2-phenylpiperidine;
(2S,3S)-(6-methoxy-2-methyl-benzothiazol-5-ylmethyl)-(2-phenylpiperidin-3-yl)amine;
(2S,3S)-[5-(2,5-dimethyl-pyrrol-1-yl)-2-methoxybenzyl]-(2-phenylpiperidin-3-yl)amine;
(2S,3S)-3-[2-methoxy-5-(5-oxazolyl)benzyl]amino-2-phenylpiperidine;
(2S,3S)-(6-methoxy-2-methyl-benzoxazol-5-ylmethyl)-(2-phenyl-piperidin-3-yl)-amine;
(1SR,2SR,3SR,4RS)-3-[6-methoxy-3-methylbenzisoxazol-5-yl]methylamino-2-benzhydrylazanorbormane;
(2S,3S)-N-(2-methoxy-5-methylsulfonylphenyl)-methyl-2-diphenylmethyl-1-azabicyclo[2.2.2]octan-3-amine;
(2S,3S)-N-(2-methoxy-5-methylthiophenyl)methyl-2-diphenylmethyl-1-azabicyclo[2.2.2]octan-3-amine;
(2S,3S)-N-(2-methoxy-5-dimethylaminophenyl)methyl-2-diphenylmethyl-azabicyclo[2.2.2]octan-3-amine; and
(2S,3S)-N-(5-trifluoroacetylamino-2-methoxyphenyl)methyl-2-diphenylmethyl-azabicyclo-[2.2.2]octan-3-amine.
(2S,3S)-3-(5-tert-butyl-2-methoxybenzyl)amino-2-(3-trifluoromethoxyphenyl)piperidine;
(2S,3S)-3-(2-isopropoxy-5-trifluoromethoxybenzyl)amino-2-phenylpipendine;
(2S,3S)-3-(2-ethoxy-5-trifluoromethoxybenzyl)amino-2-phenylpiperidine;
(2S,3S)-3-(2-methoxy-5-trifluoromethoxybenzyl)-amino-2-phenylpiperidine;
(2S,3S)-3(-5-tert-butyl-2-trifluoromethoxybenzyl)amino-2-phenylpiperidine;
2-(diphenylmethyl)-N-(2-methoxy-5-trifluoromethoxy-phenyl)methyl-azabicyclo[2.2.2]octan-3-amine;
(2S,3S)-3-[5-chloro-2-(2,2,2-trifluoroethoxy)-benzyl]amino-2-phenylpiperidine;
(2S,3S)-3-(5-tert-butyl-2-trifluoromethoxybenzyl)amino-2-phenylpiperidine;
(2S,3S)-3-(2-isopropoxy-5-trifluoromethoxybenzyl)amino-2-phenylpiperidine;
(2S,3S)-3-(2-difluoromethoxy-5-trifluoromethoxybenzyl)-amino-2-phenylpiperidine;
(2S,3S)-2-phenyl-3-[2-(2,2,2-trifluoroethoxybenzyl)-aminopiperidine; and
(2S,3S)-2-phenyl-3-(2-trifluoromethoxybenzyl)]aminopiperidine;
cis-3-(2-chlorobenzylamino)-2-phenylpiperidine;
cis-3-(2-trifluoromethylbenzylamino)-2-phenylpiperidine;
cis-3-(2-methoxybenzylamino)-2-(2-fluorophenyl)-piperidine;
cis-3-(2-methoxybenzylamino)-2-(2-chlorophenyl)-piperidine;
cis-3-(2-methoxybenzylamino)-2-(2-methylphenyl)-piperidine;
cis-3-(2-methoxybenzylamino)-2-(3-methoxyphenyl)-piperidine;
cis-3-(2-methoxybenzylamino)-2-(3-fluorophenyl)-piperidine;
cis-3-(2-methoxybenzylamino)-2-(3-chlorophenyl)-piperidine;
cis-3-(2-methoxybenzylamino)-2-phenylpiperidine;
cis-3-(2-methoxybenzylamino)-2-(3-methylphenyl)-piperidine;
cis-3-(2-methoxybenzylamino)-2-(4-fluorophenyl)-piperidine;
cis-3-(2-methoxybenzylamino)-2-(3-thienyl)-piperidine;
cis-3-(2-methoxybenzylamino)-2-phenylazacyclo-heptane;
3-(2-methoxybenzylamino)4-methyl-2-phenylpiperidine;
3-(2-methoxybenzylamino)-5-methyl-2-phenylpiperidine;
3-(2-methoxybenzylamino)-6-methyl-2-phenylpiperidine;
(2S,3S)-3-(2-methoxybenzylamino)-2-phenylpiperidine;
(2S,3S)-1-(5-carboethoxypent-1-yl)-3-(2-methoxybenzyl-amino)-2-phenylpiperidine;
(2S,3S)-1-(6-hydroxy-hex-1-yl)-3-(2-methoxybenzyl-amino)-2-phenylpiperidine;
(2S,3S)-1-(4-hydroxy-4-phenylbut-1-yl)-3-(2-methoxy-benzylamino)-2-phenylpiperidine;
(2S,3S)-1-(4-oxo-4-phenylbut-1-yl)-3-(2-methoxybenzyl-amino)-2-phenylpiperidine;
(2S,3S)-1-(5,6-dihydroxyhex-1-yl)-3-(2-methoxybenzyl-amino)-2-phenylpiperidine; cis-3-(5-fluoro-2-methoxybenzylamino)-2-phenylpiperidine;
(2S,3S)-1-[4-(4-fluorophenyl)-4-oxobut-1-yl]-3-(2-methoxybenzylamino)-2-phenylpiperidine;
(2S,3S)-1-[4-[4-fluorophenyl)-4-hydrnxybut-1-yl]-3-(2-methoxybenzylamino)-2-phenylpiperidine;
cis-3-(2-methoxy-5-methylbenzylamino)-2-phenylpiperidine;
(2S,3S)-1-(4-benzamidobut-1-yl)-3-(2-methoxybenzyl-amino)-2-phenylpiperidine;
cis-3-(2-methoxynaphth-1-ylmethylamino)-2-phenylpiperidine;
(2S,3S)-3-(2-methoxybenzylamino)-1-(5-N-methyl-carboxamidopent-1-yl)-2-phenylpiperidine;
(2S,3S)-1-(4-cyanobut-1-yl)-3-(2-methoxybenzylamino)-2-phenylpiperidine;
(2S,3S)-1-[4-(2-naphthamido)but-1-yl]-3-(2-methoxy-benzylamino)-2-phenylpiperidine;
(2S,3S)-1-(5-benzamidopent-1-yl)-3-(2-methoxybenzyl-amino)-2-phenylpiperidine;
(2S,3S)-1-(5-aminopent-1-yl)-3-(2-methoxybenzylamino)-2-phenylpiperidine;
(2S,3S)-3-(5-chloro-2-methoxybenzylamino)-2-phenylpipendine;
(2S,3S)-3-(2,5-dymethoxybenzylamino)-2-phenylpiperidine;
cis-3-(3,5-difluoro-2-methoxybenzylamino)-2-phenylpiperidine;
cis-3-(4,5-difluoro-2-methoxybenzylamino)-2-phenylpiperidine;
cis-3-(2,5-dimethoxybenzylamino)-1-[4-(4-fluorophenyl)-4-oxobut-1-yl]-2-phenylpiperidine;
cis-3-(5-chloro-2-methoxybenzylamino)-1-(5,6-dihydroxyhex-1-yl)-2-phenylpiperidine;
cis-1-(5,6-dihydroxyhex-1-yl)-3-(2,5dimethoxy-benzylamino)-2-phenylpiperidine.
cis-2-phenyl-3-[-2(prop-2-yloxy)benzylamino]piperidine;
cis-3-(2,5-dimethoxybenzyl)amino-2-(3-methoxy-phenyl)piperidine hydrochloride;
cis-3-(5-chloro-2-methoxybenzyl)amino-2-(3-methoxy-phenyl)piperidine dihydrochloride;
cis-3-(5-chloro-2-methoxybenzyl)amino-2-(3-chloro-phenyl)piperidine dihydrochloride;
3-(2-methoxybenzylamino)-2,4-diphenylpiperidine;
cis-3-(2-methoxybenzylamino)-2-phenylpyrrolidine;
(2S,3S)-3-(5-ethyl-2-methoxybenzyl)amino-2-phenylpiperidine;
(2S,3S)-3-(5-n-butyl-2-methoxybenzyl)amino-2-phenylpiperidine;
(2S,3S)-3-(2-methoxy-5-n-propylbenzyl)amino-2-phenylpiperidine;
(2S,3S)-3-(5-isopropyl-2-methoxybenzyl)amino-2-phenylpiperidine; (2S,3S)-3-(5-s-butyl-2-methoxybenzyl)amino-2-phenylpiperidine;
(2S,3S)-3-(5-t-butyl-2-methoxybenzyl)amino-2-phenylpiperidine;
(2S,3S)-3-(2-methoxy-5-phenylbenzyl)amino-2-phenylpiperidine;
(2S,3S)-N-(5-isopropyl-2-methoxyphenyl)methyl-2-diphenylmethyl-1-azabicyclo-[2.2.2]octan-3-amine;
(2S,3S)-N-(5-tert-butyl-2-methoxyphenyl)methyl-2-diphenylmethyl-1-azabicyclo[2.2.2]octan-3-amine;
(2S,3S)-N-(5-methyl-2-methoxyphenyl)methyl-2-diphenylmethyl-1-azabicyclo[2.2.2]octan-3-amine;
(2S,3S)-N-(5-ethyl-2-methoxyphenyl)methyl-2-diphenylmethyl-azabicyclo[2.2.2]octan-3-amine;
(2S,3S)-N-(5-isopropyl-2-methoxyphenyl)methyl-2-diphenylmethyl-1-azabicyclo[2.2.2]octan-3-amine;
(2S,3S)-N-(5-sec-butyl-2-methoxyphenyl)methyl-2-diphenylmethyl-1-azabicyclo[2.2.2]octan-3-amine; and
(2S,3S)-N-(5-n-propyl-2-methoxyphenyl)methyl-2-diphenylmethyl-1-azabicyclo[2.2.2]octan-3-amine;
2,4-dimethylthiazole-5-sulfonicacid[4-methoxy-3-((2S,3S)-2-phenylpiperidin-3-ylaminomethyl)phenyl]-methylamide;
N-(4,5-dimethylthiazol-2-yl)-N-[4-methoxy-3-((2S,3S)-2-phenylpiperidin-3-yl-aminomethyl)phenyl]-methanesulfonamide;
{5-[(4,5-dimethylthiazol-2-yl)methylamino]-2-methoxybenzyl)-((2S,3S)-2-phenylpiperidin-3-yl)amine;
{5-(4,5-dimethylthiazol-2-ylamino)-2-methoxybenzyl}-((2S,3S)-2-phenylpiperidin-3-ylamine;
4,5-dimethylthiazole-2-sulfonic acid methyl-[3-((2S,3S)-2-phenylpiperidin-3-ylaminomethyl)-4-trifluoromethoxyphenyl]-amide;
2,4-dimethylthiazole-5-sulfonicacid[4-isopropoxy-3-((2S,3S)-2-phenylpiperidin-3-ylaminomethyl)phenyl]-methylamide;
2,4-dimethylthiazole-5-sulfonicacid[4-isopropoxy-3-((2S,3S)-2-phenylpipendin-3-ylaminomethyl)phenyl]-isopropylamide;
2,4-dimethylthiazole-5-sulfonic acid [4-methoxy-3-((2S,3S)-2-phenylpiperidin-3-ylaminomethyl)phenyl]-isopropylamide;
2,4-dimethylthiazole-5-sulfonic acid [4-methoxy-3-((2S,3S)-2-phenylpiperidin-3-ylaminomethyl)phenyl]-isobutylamide;
2,4-dimethylthiazole-5-sulfonicacid[4-isopropoxy-3-((2S,3S)-2-phenylpiperidin-3-ylaminomethyl)phenyl]-isobutylamide;
(3R,4S,5S,6S)-N,N-diethyl-5-(5-isopropyl-2-methoxy-benzylamino)-6-diphenylmethyl-1-azabicyclo[2.2.2]octane-3-carboxamide;
(3R,4S,5S,6S)-N,N-diethyl-5-(2,5-dimethoxybenzylamino)-6-diphenylmethyl-1-azabicyclo[2.2.2]octane-3-carboxamide;
(3R,4S,5S,6S)-5-(5-isopropyl-2-methoxybenzylamino)-6-diphenylmethyl-1-azabicyclo[2.2.2]octane-3-carboxylic acid;
(3R,4S,5S,6S)-5-(2-methoxy-2-methylthiobenzylamino)-6-diphenylmethyl-azabicyclo[2.2.2]octane-3-carboxylic acid;
(3R,4S,5S,6S)-5-(2,5-dimethoxybenzylamino)-6-diphenyl-methyl-1-azabicyclo-[2.2.2]octane-3-carboxylic acid;
(3R,4S,5S,6S)-5-(2-methoxy-5-methylbenzylamino)-6-diphenylmethyl-1-azabicydo[2.2.2]octane-3-carboxylic acid;
(3R,4S,5S,6S)-5-(5-ethyl-2-methoxybenzylamino)-6-diphenylmethyl-1-azabicyclo(2.2.2]octane-3-carboxylic acid;
(3R,4S,5S,6S)-5-(2-methoxyl-5-n-propylbenzylamino)-6-diphenylmethyl-1-azabicyclo[2.2.2]octane-3-carboxylic acid;
(3R,4S,5S,6S)-5-(5-sec-butyl-2-methoxybenzylamino)-6-diphenylmethyl-1-azabicyclo[2.2.2]octane-3-carboxylic acid;
(3R,4S,5S,6S)-5-(5-N-methyl-methanesulfonylamino-2-methoxy-benzylamino)-6-diphenylmethyl-1-azabicyclo[2.2.2]octane-3-carboxylic acid;
(3R,4S,5S,6S)-5-(2-methoxy-5-methylsulfinylbenzyl-amino)-6-diphenylmethyl-1-azabicyclo[2.2.2]octane-3-carboxylic acid;
(3R,4S,5S,6S)-5-(2-methoxy-5-trifluoromethoxybenzyl-amino)-6-diphenylmethyl-1-azabicyclo[2.2.2]octane-3-carboxylic acid;
(3R,4S,5S,6S)-5-(2-methoxy-5-methylsulfonylbenzyl-amino)-6-diphenylmethyl-1-azabicyclo[2.2.2]octane-3-carboxylic acid;
(3R,4S,5S,6S)-5-(5-dimethylamino-2-methoxybenzylamino)4-diphenylmethyl-1-azabicyclo[2.2.2]octane-3-carboxylic acid;
(3R,4S,5S,6S)-5-(5-isopropyl-2-methoxybenzylamino)-6-diphenylmethyl-1-azabicyclo[2.2.2]octane-2-carboxylic acid;
(3R,4S,5S,6S)-5-(2-methoxy-5-methylthiobenzylamino)-6-diphenylmethyl-1-azabycyclo[2.2.2]octane-2-carboxylic acid;
(3R,4S,5S,6S)-5-(2,5-dimethoxybenzylamino)-6-diphenylmethyl-1-azabicyclo[2.2.2]octane-2-carboxylic acid;
(3R,4S,5S,6S)-5-(2-methoxy-5-methylbenzylamino)-6-diphenylmethyl-1-azabicyclo[2.2.2]octane-2-carboxylic acid;
(3R,4S,5S,6S)-5-(5-ethyl-2-methoxybenzylamino)-6-diphenylmethyl-1-azabicyclo[2.2.2]octane-2-carboxylic acid;
(3R,4S,5S,6S)-5-(2-methoxyl-5-n-propylbenzylamino)-6-diphenylmethyl-1-azabicyclo[2.2.2]octane-2-carboxylic acid;
(3R,4S,5S,6S)-5-(5-sec-butyl-2-methoxybenzylamino)-6-diphenylmethyl-1-azabicyclo[2.2.2]octane-2-carboxylic acid;
(3R,4S,5S,6S)-5-(5-N-methylmethanesulfonylamino-2-methoxybenzyl-amino)-6-diphenylmethyl-1-azabicyclo[2.2.2]octane-2-carboxylic acid;
(3R,4S,5S,6S)-5-(2-methoxy-5-methylsulfinylbenzyl-amino)-6-diphenylmethyl-1-azabicyclo[2.2.2]octane-2-carboxylic acid;
(3R,4S,5S,6S)-5-(2-methoxy-5-trifluoromethoxybenzyl-amino)4-diphenylmethyl1-azabicyclo[2.2.2]octane-2-carboxylic acid;
(3R,4S,5S,6S)-5-(2-meth oxy-5-methylsulfonylbenzyl-amino)-6-diphenylmethyl-1-azabicyclo[2.2.2]octane-2-carboxylic acid;
(3R,4S,5S,6S)-5-(5-dimethylamino-2-methoxybenzylamino)-6-diphenylmethyl-1-azabicyclo[2.2.2]octane-2-carboxylic acid;
(3R,4S,5S,6S)-N-carbamoylmethyl-5-(5-isopropyl-2-methoxybenzylamino)-6-diphenylmethyl-1-azabicyclo[2.2.2]octane-3-carboxamide;
(3R,4S,5S,6S)-N-carboxymethyl-5-(5-isopropyl-2-methoxybenzylamino)-6-diphenylmethyl-1-azabicyclo[2.2.2]octane-3-carboxamide;
(3R,4S,5S,6S)-3-(2-carbamoylpyrrolidin-1-yl)carbonyl-5-(5-isopropyl-2-methoxybenzylamino)-6-diphenylmethyl-1-azabicyclo[2.2.2]octane;
(3R*,4S*,5S*,6S*)-N-(1-carbamoyle thyl)-5-(5-isopropyl-2-methoxybenzylamino)-6-diphenylmethyl-1-azabicyclo[2.2.2]octane-3-carboxamide;
(3R,4S,5S,6S)-N-(1-carbamoyl-3-methylbutyl)-5-(5-isopropyl-2-methoxybenzylamino)-6-diphenylmethyl-1-azabicyclo[2.2.2]octane-3-carboxamide;
(3R,4S,5S,6S)-N-(2-carbamoylethyl)-5-(5-isopropyl-2-methoxybenzylamino)-6-diphenylmethyl-1-azabicyclo[2.2.2]octane-3-carboxamide;
(2S,3S)-N-(5-isopropenyl-2-methoxyphenyl)methyl-2-diphenylmethyl-1-azabicyclo[2.2.2]octan-3-amine;
(2S,3S)-N-(2-methoxy-5-vinylphenyl)methyl-2-diphenylmethyl-1-azabicyclo[2.2.2]octan-3-amine;
(2S,3S)-N-(2-methoxy-4,5-dimethylphenyl)methyl-2-diphenylmethyl-1-azabicyclo[2.2.2]octan-3-amine;
(2S,3S)-N-(5,6,7,8-tetrahydro-3-methoxy-2-naphthyl)methyl-2-diphenylmethyl-1-azabicyclo[2.2.2]octan-3-amine;
(2S,3S)-N-(5-methoxyindan-6-yl)methyl-6-diphenylmethyl-1-azabicyclo[2.2.2]octan-3-amine;
(2S,3S)-3-(2,4-dimethoxy-5-ethylbenzylamino)-2-diphenylmethyl-1-azabicyclo[2.2.2.]octane;
(2S,3S)-2-diphenylmethyl-N-[2-methoxy-5-(diethylphosphoryl)phenyl]methyl-1-azabicyclo[2.2.2]octan-3-amine;
(3R,4S,5S,6S)-5-(5-isopropenyl-2-methoxybenzyl-amino)-6-diphenylmethyl-1-azabicyclo[2.2.2]octane-3-carboxamide;
(3R,4S,5S,6S)-6-diphenylmethyl-5-(2-me thoxy-5-methylsulfonylbenzylamino)-1-azabicyclo[2.2.2]octane-3-carboxamide;
(3R,4S,5S,6S)-5-[5-(N-acetyl-N-methylamino)-2-methoxybenzylamino]-6-diphenylmethyl-1-azabicyclo[2.2.2]octane-3-carboxamide;
(3R,4S,5S,6S)-6-diphenylmethyl-5-[2-methoxy-5-(N-methyl-N-methylsulfonylamino)benzylamino]-1-azabicyclo[2.2.2]octane-3-carboxamide;
(3R,4S,5S,6S)-6-diphenylmethyl-5-(2-me thoxy-5-methylsulfonylbenzylamino)-1-azabicyclo[2.2.2]octane-3-carboxylic acid;
(2S,3S)-N-[5-(1-hydroxy-1-methylethyl)-2-methoxy-phenyl]methyl-2-diphenylmethyl-1-azabicyclo[2.2.2]octan-3-amine;
(2S,3S)-N-[2-methoxy-5-(1-methoxy-1-methylethyl)-phenyl]methyl-2-diphenylmethyl-1-azabicyclo[2.2.2]octan-3-amine;
(3R,4S,5S,6S)-3-[5-(1-hydroxy-1-methylethyl)-2-methoxyphenyl]methylamino-6-diphenylmethyl-1-azabicyclo[2.2.2]octan-5-carboxylic acid;
(2S,3S)-2-diphenylmethyl-N-[5-(1-hydroxy-1-hydroxymethylethyl)-2-methoxyphenyl]methyl-1-azabicyclo[2.2.2]octan-3-amine;
(3R,4S,5S,6S)-3-[5-(1-methoxy-1-methylethyl)-2-methoxyphenyl]methylamino-6-diphenylmethyl-1-azabicyclo[2.2.2]octan-5-carboxylic acid;
(3R,4S,5S,6S)-3-[5-(1-hydroxy-1-methylethyl)-2-methoxyphenyl]methylamino-6-diphenylmethyl-1-azabicyclo[2.2.2]octan-5-carboxylic acid;
(3R,4S,5S,6S)-3-[5-(1-ethylthio-1-methylethyl)-2-methoxyphenyl]methylamino-6-diphenylmethyl-1-azabicyclo[2.2.2]octan-5-carboxylic acid;
(3S,4R,5S,6S)-N-carbamoylmethyl-6-diphenylmethyl-5-(3,5-bis(trifluoromethyl)benzyloxy)-1-azabicyclo[2.2.2] octane-3-carboxamide;
(3S,4R,5S,6S)-6-diphenylmethyl-5-(3,5-bis(trifluoromethyl)benzyloxy)-1-azabicyclo[2.2.2]octane-3-carboxamide;
(3S,4R,5S,6S)-N,N-(3-oxa-1,5-pentylene)-6-diphenylmethyl-5-(3,5-bis(trifluoromethyl)benzyloxy)-1-azabicyclo[2.2.2]octane-3-carboxamide;
(3S,4R,5S,6S)-6-diphenylmethyl-5-(3,5-dimethylbenzyloxy)-1-azabicyclo[2.2.2]octane-3-carboxylic acid;
(3S,4R,5S,6S)-N,N-diethyl-6-diphenylmethyl-5-(3,5-bis(trifluoromethyl)benzyloxy)-1-azabicyclo[2.2.2]octane-3-carboxamide;
(3S,4R,5S,6S)-6-diphenylmethyl-5-(3-fluoro-5-trifluoromethylbenzyloxy)-1-azabicyclo[2.2.2]octane-3-carboxylic acid;
(3S,4R,5S,6S)-6-diphenylmethyl-5-(3,5-bis(trifluoromethyl)benzyloxy)-azabicyclo[2.2.2]octane-3-carboxylic acid;
(3S,4R,5S,6S)-N,N-dimethyl-6-diphenylmethyl-5-(3,5-bis(trifluoromethyl)benzyloxy)-1-azabicyclo[2.2.2]octane-3-carboxamide;
(2S*,3S*,4S*,5R*)-4-carboxy-3-[N-(5-isopropyl-2-methoxybenzyl)amino]-5-methyl-2-phenylpyrrolidine;
(2S*,3S*,5S*)-5-carboxy-3-[N-(2-methoxy-5-trifluoromethoxybenzyl)amino]-2-phenylpiperidine;
(2S,3S)-3-(6-methoxy-1-methyl-2-oxo-1,2,3,4-tetrahydroquinolin-7-yl)methyl-2-phenylpiperidine-3-amine;
(2S,3S)-2-phenyl-N-[5-[2,2,2-trifluoro-1-(trifluoromethyl)ethyl]-2-methoxybenzyl]piperidine-3-amine;
(2S,3S)-2-diphenylmethyl-N-[2-methoxy-5-(methylsulfonyl)benzyl]-1-azabicyclo[2.2.2]ctane-3-amine;
(2S,3S)-2-diphenylmethyl-N-(5-isopropenyl-2-methoxybenzyl)-1-azabicyclo[2.2.2]octane-3-amine; and
(2S,3S)-2-diphenylmethyl-N-[5-(1-hydroxy-1-methylethyl)-2-methoxybenzyl]-1-azabicyclo[2.2.2]octane-3-amine.
